(19) **Europäisches Patentamt**
**European Patent Office**
**Office européen des brevets**

(11) Publication number: **0 407 092 A1**

## EUROPEAN PATENT APPLICATION

(21) Application number: 90307053.0

(22) Date of filing: 27.06.90

(51) Int. Cl.⁵: **C07K 15/06, C12N 5/02, C07K 15/00, C12N 5/00**

(30) Priority: 30.06.89 JP 169019/89

(43) Date of publication of application:
09.01.91 Bulletin 91/02

(84) Designated Contracting States:
AT BE CH DE DK ES FR GB GR IT LI LU NL SE

(71) Applicant: **Takahashi, Naommy**
**2-9, Sakurajosui 5-chome, Setagaya-ku Tokyo-to(JP)**

Applicant: **FOUNDATION OF MAYEKAWA HOONKAI**
**13-1, Botan 2.chome, Koto-ku Tokyo-to(JP)**

(72) Inventor: **Takahashi, Naommy**
**2-9, Sakurajosui 5-chome Setagaya-ku Tokyo-to(JP)**

(74) Representative: **Allard, Susan Joyce et al**
**BOULT, WADE & TENNANT, 27 Furnival Streeteet**
**London EC4A 1PQ(GB)**

(54) Lymphocyte-activating factor, method for the preparation thereof and method for the proliferation of antibody-forming lymphocytes and production of antibody using the same.

(57) A novel lymphocyte-activating factor having an activity for stimulating proliferation and antibody-production of lymphocytes can be derived from small-intestine mucosal epithelia of a mammalian animal such as cattle by mashing the mucosal epithelia, removing the solid matter from the mush, dialyzing the extract, and precipitating the effective fraction in the undialyzed extract by salting-out. The lymphocyte-activating factor can be used for the production of an antibody against pathogenic viruses by culturing lymphocytes inoculated with the virus in a medium containing the factor.

# LYMPHOCYTE-ACTIVATING FACTOR, METHOD FOR THE PREPARATION THEREOF AND METHOD FOR THE PROLIFERATION OF ANTIBODY-FORMING LYMPHOCYTES AND PRODUCTION OF ANTIBODY USING THE SAME

The present invention relates to a novel lymphocyte-activating factor. More particularly, the invention relates to a novel lymphocyte-activating factor capable of stimulating proliferation of lymphocytes without losing the antibody-forming activity as well as a method for the preparation thereof and a method for the proliferation of antibody-forming lymphocytes and production of antibody using the same.

Along with the rapid progress in the biotechnology in recent years, a vital reaction modulator or a so-called biological response modifier (BRM) , which enhances the protective power of a living body against foreign bodies by acting on the immunological mechanism of the body, is highlighted in relation to the therapy of cancers. It is known that cancer cells, being a kind of foreign body in the living body, proliferate sequentially when there is a decrease in the protecting power of the living body or when a cancer cell subtlely escapes the foreign-body surveillance of the body.

Accordingly, there is a possibility that cancer cells, as a foreign body could be eliminated when measures are taken to enhance the protective function of the living body including the above mentioned immunological mechanism and others. The above mentioned BRM is a substance capable of enhancing the protective function of a living body against foreign bodies. Various factors are reported to have activity as a BRM, of which the lymphokine as a secretion of lymphocytes, cytokine as a secretion of various cells in a living body and monokine produced by macrophages are examples known in recent years.

Animals in general have an immunological mechanism which means that, when invasion of pathogenic microorganisms or other foreign bodies into the living body takes place, the immunocompetent cells act to eliminate the foreign bodies so as to maintain the metabolic system of the living body in a normal condition. Lymphocytes are the most popular ones among the immunocompetent cells and include so-called B-cells and T-cells. The B-cells exhibit a function to produce various kinds of antibodies by means of which the foreign bodies invading the living body are neutralized or detroxicated while the T-cells adequately control the antibody-producing activity of the B-cells.

Known immunocompetent cells include natural killer cells, which directly kill the malignant bodies, and macrophages, which capture and phagocytize the bacteria or foreign bodies.

Various factors have been known hitherto which are capable of activating lymphocytes so as to stimulate proliferation and differentiation of lymphocytes including interleukin-1 (IL-1) ,capable of stimulating proliferation of T-cells and differentiation of B-cells, interleukin-2 (IL-2), capable of stimulating proliferation of the T-cells and enhancing the secretion of lymphokines such as $\tau$-interferon, interleukin-4 (IL-4) capable of stimulating proliferation of the B-cells, interleukin-5 (IL-5),capable of inducing ultimate differentiation of the B-cells into antibody-producing cells, interleukin-6 (IL-6), as a differentiating factor of the B-cells to promote production of antibodies though without stimulation for the proliferation of the B-cells, and the like.

A problem or disadvantage in these hitherto known cell-differentiation stimulating factors, however, is that their activity on the B-cells, T-cells and macrocells is specific and, in addition, the activity, for example, for the stimulation of the antibody production is gradually decreased as the differentiation of the cells proceeds.

The present invention accordingly has an object to provide a novel lymphocyte-activating factor which is effective on any of the B-cells, T-cells and macrocells irrespective of the cell type and capable of stimulating both of cell proliferation and antibody production by overcoming the above described problems and disadvantages in the conventional cell-activating factors.

Thus, the cell-activating factor provided by the invention is a lymphocyte-activating factor derived from small-intestinal mucosal epithelia of a mammalian animal for use in stimulating proliferation and antibody production of lymphocytes.

The above defined lymphocyte-activating factor of the invention can be prepared by a method comprising the steps of:

(a) mashing small-intestinal mucosal epithelia of a mammalian animal to give a mush;

(b) removing the solid matter from the mush to give an extract of the small intestinal mucosal epithelium;

(c) subjecting the extract to dialysis and obtaining the undialyzed fraction and optionally;

(d) subjecting the undialyzed fraction of the extract to a salting-out treatment to give precipitates; and

(e) recovering the precipitates.

Further, the method of the invention for the production of an antibody against a virus or other antigens comprises culturing lymphocytes infected with the antigen in a culture medium containing the above defined lymphocyte-activating factor.

The above described novel lymphocyte-activating factor has been obtained as a reslt of the extensive investigations undertaken by the inventor with an object to develop a novel cell-activating factor capable of stimulating differentiation of lymphocytes arriving at a discovery that the small-intestinal mucosal epithelium of a mammalian animal contains a highly active lymphocyte-activating factor which stimulates differentiation of lymphocytes and, quite unexpectedly, also stimulates antibody production of the lymphocytes. Accordingly, the inventor got the idea that an efficient method could be obtained for the production of an antibody against various kinds of pathogenic viruses such as the AIDS virus, hepatitis B virus, influenza virus and the like by utilizing the above described lymphocyte-activating factor.

The above defined lymphocyte-activating factor of the invention may be prepared by a method comprising the steps of:

(a) mashing small-intestinal mucosal epithelia of a mammalian animal by using a suitable machine such as a homogenizer to give a mush;

(b) removing the solid matter from the mush, for example, by ultracentrifuging to give an extract solution of the small-intestinal mucosal epithelium;

(c) subjecting the extract solution to dialysis;

(d) subjecting the undialyzed fraction of the extract solution to a salting-out treatment to give precipitates; and

(e) recovering the precipitates.

The thus prepared lymphocyte-activating factor can be utilized for efficiently and rapidly producing antibodies against various kinds of viruses by conducting culture of lymphocytes inoculated with the virus according to a conventional procedure in a nutritive culture medium for anti-virus antibody production with addition of the lymphocyte-activating factor.

The lymphocyte-activating factor of the invention consists of a group of thermally stable peptides having a molecular weight in the range from about 500 to about 10,000 and thermally stable so as not to be destroyed by a heat treatment at 60 °C for 30 minutes. The lymphocyte-activating factor thus obtained from the small-intestinal mucosal epithelia of a mammalian animal is a novel material not known in the prior art and characterized by the remarkable ability to stimulate not only proliferation of lymphocytes but also production of an anti-virus antibody by the lymphocytes.

The mammalian animal from which the small-intestinal mucosal epithelia are taken and used as the starting material of the inventive lymphocyte-activating factor is not limited and includes cattle, horses, sheep, goats, pigs, rabbits, rats, mice and the like. The small intestine of the animal is taken out and the mucosal epithelium stripped off therefrom by using a razor blade and mashed, if necessary, with addition of a suitable medium such as CMF-PBS (calcium- and magnesium-free phosphate buffered saline) or the like in an up to five times volume to give a mush in the form of an aqueous suspension. The mush is then subjected to ultracentrifuge at a very high velocity of, for example, 10000 G or larger to separate the solid matter from the liquid portion. The thus obtained supernatant, which is an extract solution of the small-intestinal mucosal epithelia, is dialyzed, for example, against CMF-PBS and the undialyzed fraction of the supernatant is condensed by utilizing, for example, a collodion membrane followed by filtration according to need through a micropore filter to remove bacteria. The extract solution after the above described treatment can be used as such for the purpose of anti-virus antibody production but it is more advantageous that the effective factor contained therein is isolated in the form of precipitates by a salting-out treatment of the extract solution with addition of a suitable inorganic salt such as ammonium sulfate.

The physiological activity of the inventive lymphocyte-activating factor can be estimated by several different methods. For example, the extract from the small-intestinal mucosal epithelia is added to a suitable culture medium in which splenic lymphocytes of a mammalian animal such as rat and rabbit are cultured so as to exhibit stimulation in the proliferation of the lymphocytes. Alternatively, red blood cells of sheep (SRBC) are intravenously injected to a mammalian animal so as to cause production of an antibody by the lymphocytes thereof and then the antibody-producing lymphocytes are taken out and cultured in the same manner as above so that the lymphocyte-activating factor added to the culture medium may exhibit an activity of stimulating the differentiation of the lymphocytes by means of the proliferation of the antibody-producing lymphocytes.

Any method conventionally undertaken in culturing of animal cells can be utilized in the above mentioned procedure of culturing without particular limitations. For example, the culturing is performed at a temperature in the range from 25 to 45 °C for 1 to 7 days in an atmosphere of a gaseous mixture of 95-99% by volume of air and 5-1% by volume of carbon dioxide.

The lymphocyte-activating factor of the invention originating in small-intestinal mucosal epithelia of mammalian animals has a physiological activity to stimulate not only proliferation of lymphocytes of mammalian animals but also antibody production of the lymphocytes by stimulating differentiation of the

3

lymphocytes. Accordingly, an efficient means is obtained for stimulating proliferation of antibody-producing lymphocytes by culturing the antibody-producing lymphocytes in a culture medium containing the lymphocyte-activating factor of the invention.

The following is a description of a typical procedure for the production of an anti-virus antibody utilizing the inventive lymphocyte-activating factor according to the Mishell-Dutton's method (1967) . In the first place, splenic cells of mouse are suspended in an Eagle's basal medium and the suspension is centrifuged to precipitate the cells and discard the supernatant. A fresh portion of the Eagle's basal medium is added to the thus precipitated cells to give a cell suspension in a cell concentration of about $10 \times 10^6$ cells/ml, which is then admixed with a solution containing the inventive lymphocyte-acivating factor. The cell suspension is then inoculated with the desired antigen virus, such as the influenza virus, and culturing is conducted at a temperature of 37 °C for about 1 to 7 days under an atmosphere of the Mishell-Dutton's gaseous mixture composed of 10% by volume of carbon dioxide, 7% by volume of oxygen and 83% by volume of nitrogen. An appropriate amount of a fetal calf serum is added to the culture medium at times during the culturing period.

The extent of the proliferation of the antibody-producing lymphocytes in the above described culturing process can be determined by counting the number of the antibody-producing cells according to the plaque method. In this manner, a very rapid and efficient means is provided for the mass production of antibodies against various kinds of pathogenic viruses such as AIDS virus, hepatitis B virus, influenza virus and the like by utilizing the lymphocyte-activating factor of the invention.

The lymphocyte-activating factor of the invention can be readily prepared from small intestines of mammalian animals available in any large numbers such as cattle, horses, sheep, goats, pigs, rabbits, rats and the like and exhibits high activity for stimulating differentiation and antibody production of lymphocytes. Accordingly, the inventive lymphocyte-activating factor is particularly useful for the preparation of certain anti-virus antibodies, such as influenza vaccines, which hitherto are supplied only by a time-consuming process which does not meet the requirement for the urgent prophylactic vaccination against epidemics.

In the following, the invention is described in more detail by way of examples.

Preparation Example 1.

A sample of splenic lymphocytes of rats was prepared in the following manner. Rats under anaesthesia with Nembutal were each subjected to shaving on the abdominal skin and then bloodletting by dissecting the cervical vein followed by a disinfection treatment of the whole body using a 10% by weight solution of benzalkonium chloride. The abdominal wall of each of the thus treated rats was opened and the spleen was taken out. The spleen, after removal of the fats and connective tissues was taken in a dish containing a small volume of a RPMI 1640 medium and cut into pieces. The splenic lymphocytes were taken from these pieces of the spleen by forcible pipetting. A suspension of the thus collected splenic lymphocytes was filtered through a double nylon cloth to eliminate cell aggregates and connective tissues. The filtrate, i.e. a suspension of the splenic lymphocytes, was centrifuged for 5 minutes at a velocity of 150 G to precipitate the cells.

The splenic lymphocytes thus collected were washed with the RPMI 1640 medium and freed from the erythrocytes by re-suspending in a Tris-ammonium chloride buffer solution and standing for a while followed by weak centrifugation to precipitate the cells. The precipitated cells were washed three times with the RPMI 1640 medium and then once with an experimental medium to finish sample preparation of the splenic lymphocytes.

Preparation Example 2.

A sample of anti-SRBC (anti-sheep red blood cells) antibody-producing lymphocytes was prepared in the following manner. Thus, erythrocytes of sheep supplied by Biochemical Industry Co. were injected to the caudal vein of several male rats and, four days after the injection, the splenic lymphocytes were collected from these animals in substantially the same manner as in Preparation Example 1 to prepare a sample of the anti-SRBC antibody.

Example 1.

4

Sections of the small intestine extending from the duodenum to ileum were taken out from the abodominal cavities of cattle and the mucosal epithelia were stripped off therefrom by using a razor blade and homogenized in a CMF-PBS. The homogenate was subjected to ultracentrifugation for 30 minutes at 12000 G to give a supernatant which was further centrifuged for 10 minutes at 3000 G to be completely freed from solid matters.

The supernatant was dialyzed for 24 hours against a CMF-PBS under chilling with ice. The undialyzed fraction of the supernatant remaining in the dialyzing membrane was concentrated using a collodion membrane to have a concentration of about 5 mg protein/ml. The thus concentrated solution was freed from bacteria by passing through a Millipore Filter of 0.22 $\mu$m pores to give an extract of the small-intestine mucosal epithelium, referred to as the SIME hereinafter.

In a RPMI 1640 culture medium containing 3% by volume of the above mentioned SIME and 3% by volume of a new-born calf serum (NBCS) were suspended the splenic lymphocytes prepared in Preparation Example 1 in a concentration of $11 \times 10^5$ cells/ml and a 2 ml portion of this suspension taken in a tube was cultured for up to 7 days under conditions of 100% relative humidity at 37 °C in an atmosphere of a gaseous mixture of 5% by volume of carbon dioxide and 95% by volume of air to examine the increase in the number of the lymphocytes to give the results shown in Table 1 below which also shows the amount of DNA and concentration of glucose decreasing as the culture proceeded. The numbers of the lymphocytes given in the table are each an average for 9 tubes and the amounts of DNA in the table are given each for 2 tubes.

As a control, the same culturing test as above was repeated excepting omission of the SIME in the culture medium to give the results also shown in Table 1 . The starting cell concentration in this control test vas $12.5 \times 10^5$ cells/ml.

Table 1

|  |  | Days of culturing |  |  |  |  |
|---|---|---|---|---|---|---|
|  |  | 0 | 1 | 3 | 5 | 7 |
| Number of lymphocytes, $10^5$/tube | SIME added | 22 | 24 | 40 | 44 | 46 |
|  | Control | 25 | 22 | 18 | 13 | 9 |
| Amount of DNA, $\mu$g/2 tubes | SIME added | 10 | 14 | 16 | 18 | 21 |
|  | Control | 10 | 8 | 5 | 3 | 2 |
| Concentration of glucose, mg/dl | SIME added | 190 | 170 | 100 | 70 | 40 |
|  | Control | 190 | 188 | 186 | 188 | 199 |

The results in Table 1 clearly show that a remarkable increase was noted in the number of the lymphocytes and in the amount of DNA when the SIME was added to the culture medium along with consumption of glucose. As is known, the consumption of glucose in the medium as a result of stimulation in the glucose metabolism is correlated with blast formation, differentiation and proliferation of lymphocytes so that the decrease in the glucose concentration in the SIME-added medium can also be an indication of the proliferation of the lymphocytes.

Example 2.

The splenic lymphocytes obtained in Preparation Example 2, in which an anti-SRBC antibody had been formed, were cultured in the same manner as in Example 1 in culture media containing 3% by weight of the SIME and/or 3% by weight of the NBCS for 4 days at 37 °C to give a cell suspension in a concentration of $10^7$ cells/ml. A 0.1 ml portion of each of the cell suspensions was added to a mixture of 0.5 ml of a 10% by weight suspension of SRBC and 0.1 ml of a guinea pig compliment and the mixture was incubated in a closed Cunningham chamber at 37 °C for 1 hour followed by determination of the number of the hemolytic antibody-producing cells or plaque-forming cells (PFC) . The results were that the number of the PFC per chamber was 140 when both of the SIME and NBCS were added to the medium while the numbers were 100 and 68 when either the SIME alone or NBCS alone was added to the medium.

Example 3.

The SIME solution prepared in Example 1 was subjected to successive salting-out treatments by the addition of a saturated ammonium sulfate solution to give, firstly, 40% saturation, secondly, 55% saturation and, finally, 80% saturation each time followed by filtration to recover the precipitates. The thus obtained three fractions of the precipitates, referred to as the SIME fractions A, B and C corresponding to the 40%, 55% and 80% saturation, respectively, of ammonium sulfate, were each dissolved in water and dialyzed against distilled water to remove the ammonium sulfate followed by lyophilization.

The splenic lymphocytes obtained in Preparation Example 1 were cultured in the same manner as in Example 1 in three RPMI 1640 media each with addition of 5% by volume of a 4 mg N/ml solution of the SIME fractions A, B and C, respectively, dissolved in CMF-PBS to determine the cell numbers and the amounts of glucose consumption. The results are shown in Table 2 below. The fraction A was a group of thermally stable peptides to withstand a heat treatment at 60 °C for 30 minutes and the molecular weight thereof was about 500 to 10000 as determined by the ultrafiltration method using a Centrisart.

Table 2

|  |  | Days of culturing | | | |
|---|---|---|---|---|---|
|  |  | 0 | 1 | 3 | 7 |
| Fraction A | Numbers of lymphocytes, $10^5$/tube | 13 | 15 | 26 | 28 |
|  | Concentration of glucose, mg/dl | 189 | 162 | 158 | 134 |
| Fraction B | Numbers of lymphocytes, $10^5$/tube | 13 | 14 | 13 | 8 |
|  | Concentration of glucose, mg/dl | 189 | 196 | 192 | 190 |
| Fraction C | Numbers of lymphocytes, $10^5$/tube | 17 | 13 | 8 | 7 |
|  | Concentration of glucose, mg/dl | 189 | 188 | 192 | 191 |

**Claims**

1. A lymphocyte-activating factor for use in stimulating proliferation and antibody-production of lymphocytes, said factor being derived from small-intestine mucosal epithelia of a mammalian animal.

2. A lymphocyte-activating factor as claimed in claim 1 wherein the mammalian animal is a cow, horse, sheep, goat, pig, rabbit, rat or mouse.

3. A method for the preparation of a lymphocyte-activating factor for use in stimulating proliferation and antibody-production of lymphocytes which comprises the steps of:

(a) mashing small-intestinal mucosal epithelia of a mammalian animal to give a mush;

(b) removing the solid matter from the mush to give an extract of the small-intestinal mucosal epithelium;

(c) subjecting the extract to dialysis and obtaining the undialyzed fraction.

4. A method as claimed in claim 3 further comprising the steps of:

(d) subjecting the undialyzed fraction of the extract to a salting-out treatment to give precipitates and

(e) recovering the precipitates.

5. A method for the preparation of antibodies against an antigen which comprises culturing lymphocytes inoculated with the said antigen in a culture medium containing a lymphocyte-activating factor which stimulates proliferation and antibody-production of lymphocytes said factor being derived from small-intestine mucosal epithelia of a mammalian animal.

6. A method as claimed in claim 5 wherein said antigen is a virus.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | INTERNATIONAL JOURNAL OF TISSUE REACTIONS vol. XI, no. 1, 06 September 1989, SWITZERLAND pages 7 - 13; Takahashi,N. et al: "Stimulatory effects of small intestine mucosal factors on the proliferation and antibody formation of primarily cultured....." * the whole document * | 1-6 | C 07 K 15/06 C 12 N 5/02 C 07 K 15/00 C 12 N 5/00 |
| X | PATENT ABSTRACTS OF JAPAN vol. 10, no. 373 (C-391)(2430) 12 December 1986, & JP-A-61 166393 (MITSUBISHI CHEM IND LTD) 28 July 1986, * the whole document * | 1-6 | |
| A | CHEMICAL ABSTRACTS, vol. 111, no. 25, 18 December 1989 Columbus, Ohio, USA takahashi,N.et al: "Hepatocyte repairing factor isolation from intestinal mucous membranes" &JP-A-1143900, 06-06-1989 page 150; column 1-2; ref. no. 226033 * abstract * | 1-6 | |
| A | EP-A-0 183 253   (ZOUBECK, E.) * the whole document * | 1-6 | **TECHNICAL FIELDS SEARCHED (Int. Cl.5)** A 61 K C 07 K |

The present search report has been drawn up for all claims

| Place of search | Date of completion of search | Examiner |
|---|---|---|
| The Hague | 25 October 90 | FERNANDEZ Y BRANAS F |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document